# EUROPÄISCHE PATENTSCHRIFT

(11) **EP 1 648 318 B1**
(45) Veröffentlichungstag und Bekanntmachung des Hinweises auf die Patenterteilung: **15.04.2009**
(21) Anmeldenummer: 03817597.2
(22) Anmeldetag: 29.07.2003
(51) Int. Cl.: A61B 17/70

(54) **VORRICHTUNG ZUR FIXIERUNG EINES LÄNGSTRÄGERS MIT EINEM KNOCHENFIXATIONSELEMENT**
DEVICE FOR FIXING A LONGITUDINAL CARRIER TO A BONE FIXING ELEMENT
DISPOSITIF POUR FIXER UN SUPPORT LONGITUDINAL A UN ELEMENT DE FIXATION D'OS

(43) Veröffentlichungstag der Anmeldung: 26.04.2006
(73) Patentinhaber: Synthes GmbH, 4436 Oberdorf (CH)
(72) Erfinder: MATTHYS, Romano, CH-7235 Fideris (CH)
(74) Vertreter: Lusuardi, Werther
(86) Internationale Anmeldenummer: PCT/CH2003/000516
(87) Internationale Veröffentlichungsnummer: WO 2005/009261

(56) Entgegenhaltungen:
- WO-A-98/43551
- CH-A- 632 658
- US-A- 4 920 959
- US-A- 5 290 288
- US-A- 5 676 665

## Beschreibung

Die Erfindung bezieht sich auf eine Vorrichtung gemäss dem Oberbegriff des Patentanspruchs 1.

Mit solchen Vorrichtungen ist es möglich eine entsprechende Verbindungsstange oder einen Längsträger - wie sie in der Wirbelsäulenchirurgie Verwendung finden - über ein Knochenfixationselement, vorzugsweise eine Knochenschraube mit dem Knochen zu verbinden um ein rigides Konstrukt zu erhalten.

Aus der US-A 4920959 WITZEL ET AL. ist ein *Fixateur externe* bekannt, der relativ umständlich ist. Insbesondere müssen die einzelnen Klammerelemente auf die Längsstäbe aufgefahren werden.

Aus der CH-A 632658 ist ein Implantat zur Fixierung von Knochen bekannt, bei welchem die Qualität der Arretierung direkt von der realisierbaren Vorspannkraft zwischen Bridenkörper und Knochen abhängig ist. Auch hier müssen - im Falle einer Verwendung des Implantats als Klammer - die einzelnen Klammerelemente auf zwei parallele Längsstäbe aufgefahren werden. Ein seitliches Aufsetzen ist somit nicht möglich. Dies kann bei einer Freihandanwendung, im speziellen perkutan und minimalinvasiv, schwer realisierbar sein und einen grossen Nachteil darstellen.

Aus der US 5290288, der WO 94/01049 A1 sowie der FR 2775587 A1 sind anderseits auch offene Klammern bekannt, welche allerdings alle den Nachteil besitzen, dass sie zu Ihrer Fixation eines zusätzlichen Elementes bedürfen. Die Klammerhalterung, d.h. die Knochenschraube muss zuerst im Knochen fixiert werden und kann nicht frei wählbar perkutan eingebracht werden, um dann erst die Klammer darauf mit der Knochenschraube positionieren und fixieren zu können. Aus diesem Grunde kann auch der Längsträger erst nach erfolgtem Positionieren der Klammer auf der Knochenschraube eingebracht werden.

Aus der WO95/13754 ist schliesslich eine Klammer bekannt, bei welcher ein Längsstab in den offenen Kanal einer Klammer seitlich einführbar ist und dort mittels einer durch die Klammer hindurchführbaren Feststellschraube festklemmbar ist. Die Feststellschraube hat aber nur diese eine Funktion und ist nicht als Knochenschraube ausgebildet, welche das ganze Konstrukt am Knochen befestigen könnte. Das gleiche gilt auch für die aus der DE-A 195 34 136 bekannte Halterung.

Die aus dem Stand der Technik bekannten Vorrichtungen sind somit ganz allgemein kompliziert und wenig flexibel bezüglich ihres Anwendungsbereiches.

Hier will die Erfindung Abhilfe schaffen. Der Erfindung liegt die Aufgabe zugrunde, eine Vorrichtung zu schaffen, welche sehr einfach aufgebaut, möglichst flexibel anwendbar ist, sowie eine minimal invasive Operationstechnik erlaubt.

Die Erfindung löst die gestellte Aufgabe mit einer Vorrichtung, welche die Merkmale des Anspruchs 1 aufweist.

Die Vorteile der efindungsgemässen Vorrichtung sind vielfältig und können wie folgt beschrieben werden:
a) sie besteht nur aus einem Teil;
b) sie kann minimal invasiv angewendet werden und bedarf keiner grossen Eröffnungsstelle;
c) es ist keine Vorspannung zwischen der Vorrichtung und dem Knochen notwendig, um die Arretierung zu gewährleisten;
d) sie kann sowohl für einen *Fixateur extern* als auch für einen *Fixateur interne* verwendet werden;
e) der Längsträger kann schon vorab in die Vorrichtung eingebracht und vorgeformt werden und der Anwender muss erst dann die Knochenschrauben setzen, wenn er auch sicher deren Position weiss;
f) die Position der einzelnen Vorrichtungen ist durch den Anwendenden frei wählbar;
g) wird eine weitere Vorrichtung benötigt, kann diese einfach auf den Längsträger aufgesteckt werden, ohne diesen mühsam über die gesamte Länge des Längsträgers aufzufahren;
h) der Längsträger wird vor dem Setzen der erfindungsgemässen Vorrichtungen eingebracht und gibt so dem Chirurgen die Möglichkeit, die Schraubenpositionen einfacher bestimmen zu können;
i) es besteht die Möglichkeit, über den Längsträger die Fraktur zu reponieren;
j) der Anwender kann die einzelnen Frakturteile minimal invasiv über ein Konstrukt bestehend aus einem Längsträger und mehreren erfindungsgemässen Vorrichtungen mittels winkelstabiler Kopfverriegelungsschrauben verbinden und fixieren; und
k) die erfindungsgemässe Vorrichtung kann nicht nur im Wirbelsäulenbereich verwendet werden, sondern auch auf anderen osteosynthetischen Anwendungsgebieten.

Die erfindungsgemässe Vorrichtung erlaubt die Aufnahme eines der Aussparung entsprechenden Längsträgers und einer Standard-Kopfverriegelungsschraube. Beim Festziehen der Kopf-Verriegelungsschraube blockiert diese den Längsträger in der erfindungsgemässen Vorrichtung.

Die Bohrung in der Vorrichtung weist zweckmässigerweise einen Konuswinkel im Bereich von 5° und 25°, vorzugsweise von 8° und 15° auf.

Die in Form von Klammern realisierten erfindungsgemässen Vorrichtungen lassen sich in zwei unterschiedlichen Ausführungsformen, einem Typ A und einem Typ B realisieren. Sie unterscheiden sich darin, dass die eine von oben (Typ A) und die andere von unten (Typ B) auf den Längsträger (Verbindungsstab) aufbringbar sind. Das Verriegelungsprinzip bleibt dabei dasselbe.
Beim Typ A verjüngt sich die konisch Bohrung zum Kanal hin und die kleinere Öffnung der Bohrung liegt auf der gleichen Seite des Körpers wie der Kanal. Dadurch ergibt sich der Vorteil, dass die Vorrichtung auf den bereits montierten Längsstab seitlich aufgesteckt werden kann.
Beim Typ B verbreitert sich die konisch Bohrung zum Kanal hin und die grössere Öffnung der Bohrung liegt auf der gleichen Seite des Körpers wie der Kanal. Dadurch ergibt sich der Vorteil, dass der Längsträger in die bereits montierte Vorrichtung seitlich eingeführt werden kann.

Bei einer besonderen Ausführungsform besitzt die Vorrichtung zusätzlich ein Knochenfixationselement, vorzugsweise in Form einer Knochenschraube, mit einer zur Einführung in den Knochen geeigneten Spitze, einem Gewindeschaft und einem zur Manipulation der Schraube geeigneten Kopf. Dies erlaubt das Reponieren der Knochenfragmente via Knochenschraube.

Bei einer weiteren Ausführungsform weist der Kopf des Knochenfixationselementes ein Aussengewinde auf, welches vorzugsweise zum Innengewinde der Bohrung korrespondiert. Dies garantiert einen möglichst geringen Materialabrieb während der Fixation. Die Steigung des Aussengewindes beträgt zweckmässigerweise 0,1 bis 3,0 mm, vorzugsweise 0,25 bis 1,50 mm.

Bei einer weiteren Ausführungsform verjüngt sich der Kopf des Knochenfixationselementes gegen die Spitze hin konisch. Der sich konisch verjüngende Kopf weist zweckmässigerweise einen Konuswinkel im Bereich von 5° bis 25°, vorzugsweise von 8° bis 15° auf.

Bei einer besonderen Ausführungsform wird eine Vorrichtung nach Typ A mit einer Vorrichtung nach Typ B kombiniert. Eine solche Kombination hat den Vorteil, dass sie - zusammen mit dem behandelten Knochen - ein rigiden Rahmen bildet.

Nachstehend werden kurz eine allgemeine und eine spezielle Operationsmethode für die erfindungsgemässe Vorrichtung beschrieben.

### Allgemeine Operationsmethode:

A) Der Längsträger wird zuerst durch eine Stichinzision in den zu behandelnden Bereich des Patienten eingebracht und perkutan in die gewünschte Position geschoben;
B) Der Chirurg kann nun die Kontur des Längsträger kontrollieren und einfach korrigieren;
C) Ist die Kontur des Längsträgers in Ordnung, können die gewünschte Anzahl Klammern (erfindungsgemässe Vorrichtungen) durch entsprechende Stichinzisionen perkutan eingebracht werden und direkt seitlich auf den Stab aufgebracht werden;
D) Nun werden winkelstabile Kopfverriegelungsschrauben in die Bohrungen der Klammern eingedreht, aber noch nicht festgezogen.
E) Ist die Reposition in Ordnung, können die winkelstabilen Kopfverriegelungsschrauben definitiv festgezogen werden, so dass das aus dem Längsträger, den Klammern und den Knochenschrauben gebildete Konstrukt rigide wird und die Fraktur fixiert.

### Spezielle Operationsmethode:

1. Die erfindungsgemässe Vorrichtung (Klammer) vom Typ B wird relativ zum gewünschten Knochenteil positioniert;
2. Eine Kopfverriegelungsschraube wird durch die konische Bohrung de Klammer vom Typ B auf eine vorbestimmte Eindrehtiefe eingedreht, so dass eine Vorfixierung der Klammer erreicht wird;
3. Der Längsträger wird entsprechend den anatomischen Bedürfnissen angeformt;
4. Der Längsträger wird in den offenen Kanal der bereits vormontierten Klammer vom Typ B eingelegt;
5. Die Kopfverriegelungsschraube wird nun in der Klammer vom Typ B vollständig festgezogen, so dass eine Fixierung des Längsträgers an der Klammer erfolgt;
6. Die Fraktur wird mit einem geeigneten Repositionsinstrument über den Längsträger reponiert;
7. Die reponierten Knochenfragmente werden mittels der Klammern, wahlweise vom Typ A oder Typ B, fixiert;
8. Wahlweise Ergänzung des Knochenfixations-Konstruktes mit Klammern vom Typ A oder Typ B.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Explosionsdarstellung einer Knochenfixationsvorrichtung mit einem Längsträger, zwei unterschiedlichen Typen der erfindungsgemässen Vorrichtung (Klammern) und zwei Knochenschrauben;

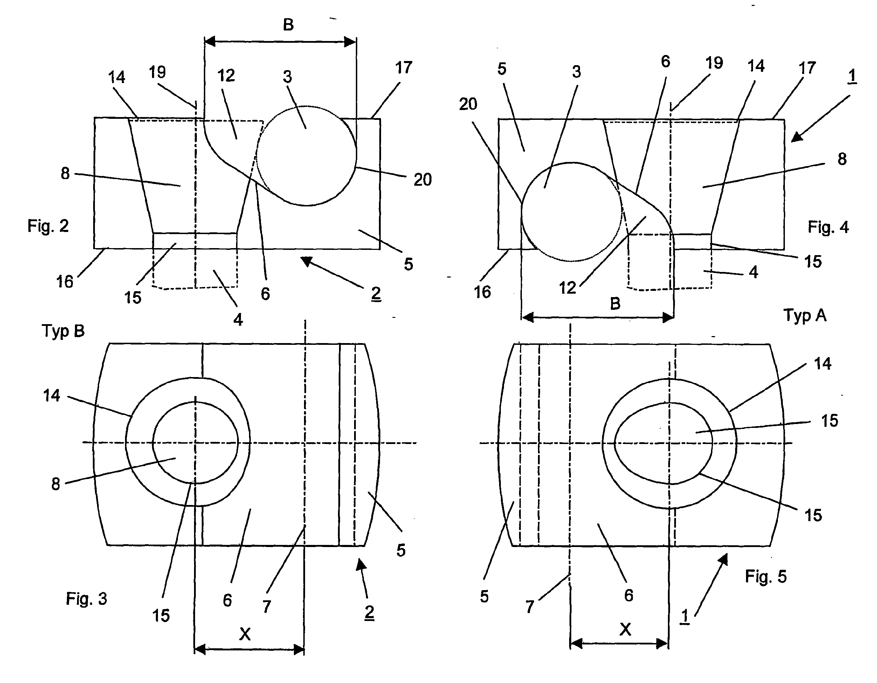

Fig. 2 eine Ansicht einer erfindungsgemässen Vorrichtung (Klammer) vom Typ B;
Fig. 3 eine Aufsicht auf die Vorrichtung nach Fig. 2;
Fig. 4 eine Ansicht einer erfindungsgemässen Vorrichtung (Klammer) vom Typ A; und
Fig. 5 eine Aufsicht auf die Vorrichtung nach Fig. 4.

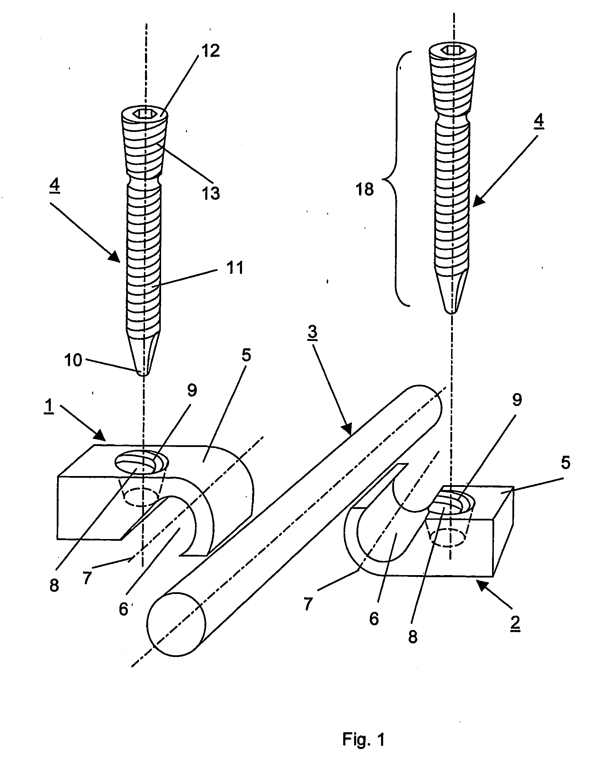

In Fig. 1 dargestellt sind zwei Ausführungsformen der Vorrichtung 1;2, welche zur Verbindung des Längsträgers 3 mit je einem Knochenfixationselement 4 dienen. Die zwei Ausführungsformen der Vorrichtung 1;2 unterscheiden sich im wesentlichen durch die Ausgestaltung des Kanals 6 zur Aufnahme des Längsträgers 3, wobei bei der ersten Ausführung (Typ A) der Kanal 6 gegen die untere, an das hier als Gewindeschaft 11 ausgebildete Segment des Knochenfixationselementes 4 gerichtete Oberfläche 16 des Körpers 5 offen ist und bei der zweiten Ausführungsform (Typ B) gegen die obere, an den Kopf 12 des Knochenfixationselementes 4 grenzende Oberfläche 17 des Körpers 5 offen ist. Die offene Oberfläche des Kanals 6 an der unteren Oberfläche 16 des Körpers 5 (Typ A), respektive an der oberen Oberfläche 17 (Typ B) weist quer zur Kanalachse 7 eine Breite B auf, welche grösser oder gleich dem Durchmesser des Längsträgers 3 ist, so dass ein Längsträger 3 quer zur Kanalachse 7 von unten (Typ A), respektive von oben (Typ B) in den Kanal 6 eingeführt werden kann.

Das Knochenfixationsmittel 4 ist bei dieser Ausführung als Knochenschraube 18 ausgestaltet und umfasst einen in einen Knochen, insbesondere einen Pedikel einschraubbaren Gewindeschaft 11 mit einer Spitze 10 und einem konischen Kopf 12, wobei das einen kleineren Durchmesser aufweisende Ende des konischen Kopfes 12 gegen den Gewindeschaft 11 gerichtet ist. Der Körper 5 wird von einer zum Kopf 12 des Knochenfixationselementes 4 komplementären, konischen Bohrung 8 von der oberen Oberfläche 17 bis zur unteren Oberfläche 16 durchdrungen, wobei die Längsachse 19 der Bohrung 8 windschief zur Kanalachse 7 steht. Bei beiden Ausführungsformen Typ A und Typ B der Vorrichtung 1;2 münden die grössere Öffnung 14 der Bohrung 8 in die obere Oberfläche 17 und die kleinere Öffnung 15 in die untere Oberfläche 16. Insbesondere für die hier dargestellten Ausführungsformen Typ A und Typ B der Vorrichtung 1;2 steht die Längsachse 19 der Bohrung 8 senkrecht zur Kanalachse 7, wobei zwischen Längsachse 19 und Kanalachse 7 ein Abstand X liegt. Dieser Abstand X ist derart bemessen, dass die konische Bohrung 8 den Kanal 6 schneidet, wobei der Abstand X grösser als die Summe von Radius R des Längsträgers 3 und Radius r des Gewindeschaftes 11 des Knochenfixationselementes 4 ist. Damit ist erreichbar, dass der Gewindeschaft 11 des Knochenfixationselementes 4 auch bei in den Kanal 6 eingeführtem Längsträger 3 durch die Bohrung 8 durchführbar ist und in einen Knochen eingeschraubt werden kann, bis der zur Bohrung 8 komplementär konische Kopf 12 des Knochenfixationselementes 4 soweit in die Bohrung 8 eingeführt ist, dass er am Längsträger 3 anliegt und durch Anziehen des Knochenfixationselement 4 eine Fixierung des Längsträgers 3 im Kanal 6 erreichbar ist.

Für beide Typen A;B der Vorrichtung 1;2 umfasst der Kanal 6 eine von der Bohrung 8 entfernte, konkave Seitenwand 20, welche einen in den Kanal 6 eingelegten Längsträger 3 gegen Verschiebungen parallel zur Längsachse 19 der Bohrung 8 sichert. Beim Anziehen des Knochenfixationselementes 4 wird der in den Kanal 6 eingelegte Längsträger 3 durch den konischen Kopf 12 quer zur Kanalachse 7 gegen die konkave Seitenwand 20 des Kanals 6 gepresst und im Kanal 6 fixiert. Ferner sind die Bohrung 8 mit einem Innengewinde 9 und der Kopf 12 des Knochenfixationselementes 4 mit einem komplementären Aussengewinde 13 versehen, so dass beim Anziehen des Knochenfixationselementes 4 eine stabile Blockierung zwischen Knochenfixationselement 4, Körper 5 und Längsträger 3 herstellbar ist.

## Patentansprüche

1. Vorrichtung (1 ;2) zur gegenseitigen Fixierung eines longitudinalen Längsträgers (3) mit einem Knochenfixationselement (4), wobei die Vorrichtung aus einem dreidimensionalen Körper (5) besteht, der einen einseitig offenen Kanal (6) mit der Kanalachse (7) zur Aufnahme des Längsträgers (3) sowie eine quer zur Kanalachse (7) verlaufende, den Körper (5) vollständig durchbohrenden Bohrung (8) besitzt, wobei sich der Kanal (6) und die Bohrung (8) mindestens teilweise durchdringen,
**dadurch gekennzeichnet, dass**
A) die Bohrung (8) konisch ausgebildet ist, mit einer grösseren Öffnung (14) und einer kleineren Öffnung (15); und
B) die Bohrung (8) ein Innengewinde (9) aufweist.

2. Vorrichtung (1;2) nach Anspruch 1, **dadurch gekennzeichnet, dass** die Bohrung (8) einen Konuswinkel im Bereich von 5° und 25° aufweist.

3. Vorrichtung (1;2) nach Anspruch 2, **dadurch gekennzeichnet, dass** die Bohrung (8) einen Konuswinkel im Bereich von 8° und 15° aufweist.

4. Vorrichtung (1;2) nach einem der Ansprüche 1 bis 3, **dadurch gekennzeichnet, dass**
A) sich die konische Bohrung (8) zum Kanal (6) hin verjüngt und
B) die kleinere Öffnung (15) der Bohrung (8) auf der gleichen Seite des Körpers (5) liegt wie der Kanal (6).

5. Vorrichtung (1 ;2) nach einem der Ansprüche 1 bis 3, **dadurch gekennzeichnet, dass**
A) sich die konische Bohrung (8) zum Kanal (6) hin verbreitert und
B) die grössere Öffnung (14) der Bohrung (8) auf der gleichen Seite des Körpers (5) liegt wie der Kanal (6).

6. Vorrichtung (1 ;2) nach einem der Ansprüche 1 bis 5, **dadurch gekennzeichnet, dass** sie zusätzlich ein Knochenfixationselement (4), vorzugsweise in Form einer Knochenschraube aufweist, mit einer zur Einführung in den Knochen geeigneten Spitze (10), einem Gewindeschaft (11) und einem zur Manipulation der Schraube geeigneten Kopf (12).

7. Vorrichtung nach Anspruch 6, **dadurch gekennzeichnet, dass** der Kopf (12) des Knochenfixationselementes (4) ein Aussengewinde (13) aufweist, welches vorzugsweise zum Innengewinde (9) korrespondiert.

8. Vorrichtung nach Anspruch 7, **dadurch gekennzeichnet, dass** die Steigung des Aussengewindes (13) zwischen 0,1 und 3,0 mm, vorzugsweise zwischen 0,25 und 1,50 mm liegt.

9. Vorrichtung nach einem der Ansprüche 6 bis 8, **dadurch gekennzeichnet, dass** der Kopf (12) des Knochenfixationselementes (4) sich gegen die Spitze (10) hin konisch verjüngt.

10. Vorrichtung nach Anspruch 9, **dadurch gekennzeichnet, dass** der sich konisch verjüngende Kopf (12) einen Konuswinkel im Bereich von 5° und 25°, vorzugsweise zwischen 8° und 15° aufweist.

11. Knochenfixationsvorrichtung mit einem Längsträger (3) und mindestens einer Vorrichtung nach Anspruch 4 sowie mindestens einer Vorrichtung nach Anspruch 5.

## Claims

1. Device (1; 2) for mutually fixing a longitudinal carrier (3) and a bone fixation element (4), the device consisting of a three-dimensional body (5), which has a channel (6) with a channel axis (7), which is open at one side for receiving the longitudinal carrier (3), as well as a borehole (8), which extends transversely to the channel axis (7) and passes through the body (5) completely, the channel (6) and the borehole (8) penetrating one another at least partly,
**characterized in that**
A) the borehole (8) is constructed conically with a larger opening (14) and a smaller opening (15) and
B) the borehole (8) has an internal thread (9).

2. The device (1; 2) of claim 1, **characterized in that** the borehole (8) has a conical angle ranging from 5° to 25°.

3. The device (1; 2) of claim 2, **characterized in that** the borehole (8) has a conical angle ranging from 8° to 15°.

4. The device (1; 2) of one of the claims in one to 3,
**characterized in that**
A) the conical borehole (8) tapers towards the channel (6) and
B) the smaller opening (15) of the borehole (8) is on the same side of the body (5) as the channel (6).

5. The device (1; 2) of one of the claims 1 to 3, **characterized in that**
A) the conical borehole (8) expands towards the channel (6) and
B) the larger opening (14) of the borehole (8) is on the same side of the body (5) as the channel (6).

6. The device (1; 2) of one of the claims 1 to 5, **characterized in that** it additionally has a bone fixation element (4), preferably in the form of a bone screw, with a tip (10), suitable for introduction into the bone, a threaded shaft (11) and a head (12) suitable for manipulating the screw.

7. The device of claim 6, **characterized in that** the head (12) of the bone fixation element (4) has an external thread (13), which preferably corresponds to the internal thread (9).

8. The device of claim 7, **characterized in that** the pitch of the external thread (13) is between 0.1 and 3.0 mm and preferably between 0.25 and 1.50 mm.

9. The device of one of the claims 6 to 8, **characterized in that** the head (12) of the bone fixation element (4) tapers conically towards the tip (10).

10. The device of claim 9, **characterized in that** the head (12), tapering conically, has a conical angle ranging from 5° to 25° and preferably from 8° to 15°.

11. Bone fixation device with a longitudinal carrier (3) and at least one device of claim 4, as well as at least one device of claim 5.

## Revendications

1. Dispositif (1 ; 2) pour la fixation mutuelle d'un élément de maintien longitudinal (3) et d'un élément de fixation osseuse (4), dans lequel le dispositif est composé d'un corps tridimensionnel (5) qui présente un canal ouvert sur un côté (6) ayant l'axe de canal (7) destiné à loger l'élément de maintien longitudinal (3) ainsi qu'un alésage (8) s'étendant transversalement à l'axe de canal (7) et traversant entièrement le corps (5), dans lequel le canal (6) et l'alésage (8) pénètrent au moins partiellement l'un dans l'autre,
**caractérisé en ce que**
A) l'alésage (8) est réalisé en forme de cône, avec une ouverture plus grande (14) et une ouverture plus petite (15) ; et
B) l'alésage (8) présente un taraudage (9).

2. Dispositif (1 ; 2) selon la revendication 1, **caractérisé en ce que** l'alésage (8) présente un angle de conicité dans la gamme de 5° à 25°.

3. Dispositif (1 ; 2) selon la revendication 2, **caractérisé en ce que** l'alésage (8) présente un angle de conicité dans la gamme de 8° à 15°.

4. Dispositif (1 ; 2) selon l'une quelconque des revendications 1 à 3,
**caractérisé en ce que**
A) l'alésage conique (8) rétrécit en direction du canal (6) et
B) l'ouverture plus petite (15) de l'alésage (8) est située sur le même côté du corps (5) que le canal (6).

5. Dispositif (1 ; 2) selon l'une quelconque des revendications 1 à 3,
**caractérisé en ce que**
A) l'alésage conique (8) s'élargit en direction du canal (6) et
B) l'ouverture plus grande (14) de l'alésage (8) est située sur le même côté du corps (5) que le canal (6).

6. Dispositif (1 ; 2) selon l'une quelconque des revendications 1 à 5, **caractérisé en ce qu'**il présente en plus un élément de fixation osseuse (4), de préférence sous la forme d'une vis à os, avec une pointe (10) appropriée à être introduite dans l'os, une tige filetée (11) et une tête (12) conçue pour la manipulation de la vis.

7. Dispositif selon la revendication 6, **caractérisé en ce que** la tête (12) de l'élément de fixation osseuse (4) présente un filetage (13) qui correspond de préférence au taraudage (9).

8. Dispositif selon la revendication 7, **caractérisé en ce que** le pas du filetage (13) est compris entre 0,1 et 3,0 mm, de préférence entre 0,25 et 1,50 mm.

9. Dispositif selon l'une quelconque des revendications 6 à 8, **caractérisé en ce que** la tête (12) de l'élément de fixation osseuse (4) rétrécit de manière conique vers la pointe (10).

10. Dispositif selon la revendication 9, **caractérisé en ce que** la tête rétrécissant de manière conique (12) présente un angle de conicité dans une gamme allant de 5° à 25°, de préférence de 8° à 15°.

11. Dispositif de fixation osseuse comprenant un élément de maintien longitudinal (3) et au moins un dispositif selon la revendication 4 ainsi qu'au moins un dispositif selon la revendication 5.
